# EUROPEAN PATENT APPLICATION

(11) **EP 2 179 728 A1**
(43) Date of publication of application: **28.04.2010**
(21) Application number: 07800919.8
(22) Date of filing: 10.08.2007
(51) Int. Cl.: A61K 9/48, A61K 47/36, A61K 47/38, A61K 47/32, A61K 47/10

(54) **NON-GELATIN SHELL MATERIAL OF HARD CAPSULE AND PROCESS FOR PREPARING THEREOF**

(71) Applicant: Shanghai Huiyuan Vegetal Capsule Co., Ltd, Shanghai 201203 (CN)
(72) Inventor: ZHANG, Shichu, Shanghai 201203 (CN); WU, Guoqing, Shanghai 201203 (CN)
(74) Representative: Grünecker, Kinkeldey, Stockmair & Schwanhäusser Anwaltssozietät
(86) International application number: PCT/CN2007/070443
(87) International publication number: WO 2009/021377

(57) **Abstract**

The invention provides a non-gelatin shell material of hard capsule, which comprises 0.01-18 parts by weight of a hydrophilic non-gelatin gelling agent, 62-96 parts by weight of an adhesive, 3-10 parts by weight of water, 0-5 parts by weight of a co-gelling agent, and 0-5 parts by weight of a plasticizer, wherein the sum of the weights of the gelling agent, the adhesive and the water is 85wt%-100wt% of the total weight of the hard capsule shell material.

## Description

### Technical Field

The invention relates to the field of material, in particular to the components of a hard capsule shell and the process for manufacturing the hard capsule shell.

### Background Art

Nowadays, the membrane of a hard capsule is typically made from gelatin extracted from such material as the bone or skin of a cattle or a pig and the like. Despite the nice characteristics exhibited by gelatin in preparing a hard capsule, gelatin as an exogenous protein may be antigenic. For example, gelatin material derived from imported cattle may contain prion virus, so that the soft capsule made using it as the raw material may lead to a diffusion of mad cow disease. Furthermore, the production of pharmaceuticals is not favored by a gelatin hard capsule which tends to be deformed or become brittle at low humidity or at high or low temperatures. It is urgent to develop a non-gelatin other than gelatin material of hard capsules along with the growing demand for non-gelatin source materials by the market. In order to overcome the foregoing disadvantages suffered by gelatin hard capsules, it is an urgent need in the art to develop a new non-gelatin shell material of hard capsule and a production process for making the same which is adapted to be produced on the same production line formerly used for a gelatin hard capsule and is able to disintegrate at a higher rate than the gelatin hard capsule.

### Summary of the Invention

One object of the invention is to provide a non-gelatin shell material of hard capsule which is disintegrable rapidly.

Another object of the invention is to provide a hard capsule which is disintegrable rapidly.

Still another object of the invention is to provide a process for manufacturing a non-gelatin shell of hard capsule according to the invention.

According to the first aspect of the invention, there is provided a non-gelatin shell material of hard capsule, comprising the following components:
(A) 0.01-18 parts by weight of a hydrophilic non-gelatin gelling agent;
(B) 62-96 parts by weight of an adhesive;
(C) 3-10 parts by weight of water;
(D) 0-5 parts by weight of a co-gelling agent; and
(E) 0-5 parts by weight of a plasticizer;
wherein the sum of the weights of components (A) + (B) + (C) is 85wt%-100wt% of the total weight of the hard capsule shell material.

In one preferred embodiment, the adhesive of the component (B) is selected from the following adhesives: starch, dextrin, polyvinyl pyrrolidone, carboxymethyl chitin, polyvinyl alcohol, sesbania gum, arabic gum, cassia gum, pullulan, pullulan acetate, elsinan, Indian gum, glucan, copolymer of vinyl pyrrolidone and vinyl acetate, hydroxyalkyl cellulose, carboxyalkyl cellulose or combinations thereof.

In one preferred embodiment, the adhesive of the component (B) is selected from starch, hydroxyalkyl cellulose or a combination thereof.

In one preferred embodiment, the starch is selected from high amylose, etherified starch, etherified high amylose, etherified high amylopectin, oxidized starch, oxidized high amylose, oxidized high amylopectin, esterified starch, esterified high amylose, esterified high amylopectin or combinations thereof; and/or

The hydroxyalkyl cellulose is selected from hydroxypropyl methyl cellulose, hydroxyethyl methyl cellulose, hydroxypropyl cellulose or combinations thereof.

In one preferred embodiment, the gelling agent of the component (A) is selected from the group consisting of the following hydrophilic gelling agents: highly acetylated gellan gum, lowly acetylated gellan gum, κ-carrageenan, τ-carrageenan, β-carrageenan, agar, pectin, sodium alginate, xanthan gum, tragacanth gum, karaya gum, locust bean gum, furcellaran gum, tamarind gum, tara gum, sclerotium glucan, microbial alginate, carbomer or combinations thereof;

The co-gelling agent of the component (D) is selected from the group consisting of the following co-gelling agents: potassium chloride, calcium chloride, potassium phosphate, potassium citrate or combinations thereof; and/or

The plasticizer of the component (E) is selected from the group consisting of the following plasticizers: glycerin, sorbitol, polyethylene glycol, ethylene glycol, ethyl acetate, 1,3-butylene glycol, 1,4-butylene glycol, xylitol, glyceryl diacetate, glyceryl triacetate, glyceryl monoacetate, mannitol, myoinositol, maltitol, glucose, polypropylene glycol or combinations thereof.

In one preferred embodiment, there is provided a hard capsule shell material according to the invention, substantially consisting of a hydrophilic gelling agent, an adhesive, an optional plasticizer, an optional co-gelling agent and water, wherein the hydrophilic gelling agent is 0.01-18 parts by weight, the adhesive is 62-96 parts by weight, the water is 3-10 parts by weight, the plasticizer is 0-5 parts by weight, the co-gelling agent is 0-5 parts by weight, and the total weight of the hydrophilic gelling agent, the adhesive and the water is 85 wt%-100 wt% of the total weight of the hard capsule shell material, and wherein:
the gelling agent is selected from the group consisting of the following hydrophilic gelling agents: highly acetylated gellan gum, lowly acetylated gellan gum, κ-earrageenan, τ-carrageenan, β-carrageenan, agar, pectin, sodium alginate, xanthan gum, tragacanth gum, karaya gum, locust bean gum, furcellaran gum, tamarind gum, tara gum, sclerotium glucan, microbial alginate, carbomer or combinations thereof;
the adhesive is selected from the following adhesives: starch, dextrin, polyvinyl pyrrolidone, carboxymethyl chitin, polyvinyl alcohol, sesbania gum, arabic gum, cassia gum, pullulan, pullulan acetate, elsinan, Indian gum, glucan, copolymer of vinyl pyrrolidone and vinyl acetate, hydroxyalkyl cellulose, carboxyalkyl cellulose or combinations thereof;
the plasticizer is selected from the group consisting of the following plasticizers: glycerin, sorbitol, polyethylene glycol and mixtures thereof, ethylene glycol, ethyl acetate, 1,3-butylene glycol, 1,4-butylene glycol, xylitol, glyceryl diacetate, glyceryl triacetate, glyceryl monoacetate, mannitol, myoinositol, maltitol, glucose, polypropylene glycol or combinations thereof; and/or
the co-gelling agent is selected from the group consisting of the following co-gelling agents: potassium chloride, calcium chloride, potassium phosphate, potassium citrate or combinations thereof.

In one preferred embodiment, the starch is selected from high amylose, etherified starch, etherified high amylose, etherified high amylopectin, oxidized starch, oxidized high amylose, oxidized high amylopectin, esterified starch, esterified high amylose, esterified high amylopectin or combinations thereof.

In one preferred embodiment, the adhesive of the component (B) is selected from starch, hydroxyalkyl cellulose or a combination thereof.

In one preferred embodiment, the starch is selected from high amylose, etherified starch, etherified high amylose, etherified high amylopectin, oxidized starch, oxidized high amylose, oxidized high amylopectin, esterified starch, esterified high amylose, esterified high amylopectin or combinations thereof; and/or

The hydroxyalkyl cellulose is selected from hydroxypropyl methyl cellulose, hydroxyethyl methyl cellulose, hydroxypropyl cellulose or combinations thereof.

According to the second aspect of the invention, there is provided a hard capsule made from the non-gelatin shell material of hard capsule according to the invention.

According to the third aspect of the invention, there is provided a process for manufacturing a non-gelatin shell of hard capsule, comprising the following steps:
(a) providing 0.01-18 parts by weight of a hydrophilic non-gelatin gelling agent, 62-96 parts by weight of an adhesive, 0-5 parts by weight of an optional co-gelling agent and 0-5 parts by weight of an optional plasticizer, and mixing the foregoing components with water to give a gel solution of the dissolved components;
(b) using the gel solution obtained in step (a) to make a non-gelatin shell of hard capsule by gel-dipping or pressing; and
(c) drying the non-gelatin shell of hard capsule obtained in step (b) at 30-45°C to give a non-gelatin shell of hard capsule containing 3-10 parts by weight of water.

### Detailed Description of the Invention

After a long term of intensive and extensive research aimed at overcoming the shortcomings of the gelatin hard capsules, the inventors have found, based on the characteristics of non-gelatin colloid material, that a hard capsule shell material with good taste and high clarity may be formed by compounding an adhesive and a hydrophilic gelling agent at certain ratios, and an ideal non-gelatin shell of hard capsule may be made therefrom. The invention is fulfilled on the basis of this finding. Furthermore, in one preferred embodiment, the inventors have found that a faster disintegrating rate and a better clarity can be achieved for the resultant capsule by using a specific mixture of starch and cellulose as the adhesive.

As used herein, the term "comprise" or "include" means various components may be used together in the mixture or composition according to the invention. Thus, the terms "mainly consisting of" and "consisting of" are covered by the term "comprise" or "include".

As used herein, unless otherwise specified, an "alkyl" refers to a linear or branched alkane containing 2-20 carbon atoms, which may include one or more carbon-carbon double bonds or triple bonds. Preferably, it refers to an alkane containing 2-10 carbon atoms, e.g. including but not limited to methyl, ethyl, n-propyl, iso-propyl, n-butyl, iso-butyl, and tert-butyl.

The various aspects according to the inventions will be described in detail as follows.

### Hydrophilic Non-gelatin Gelling Agent

Hydrophilic gelling agents useful in the invention are hydrophilic non-gelatin gelling agents without exception, representative examples of which include but are not limited to highly acetylated gellan gum, lowly acetylated gellan gum, κ-carrageenan, τ-carrageenan, β-carrageenan, agar, pectin, sodium alginate, xanthan gum, tragacanth gum, karaya gum, locust bean gum, furcellaran gum, tamarind gum, tara gum, sclerotium glucan, microbial alginate, carbomer or combinations thereof.

Hydrophilic gelling agents according to the invention are all commercially available compounds, the molecular weights of which are not limited as long as the object of the invention is not restricted thereby.

The highly acetylated gellan gum according to the invention is a commercially available compound having an acetylation degree that is typical in the art. For example, the highly acetylated gellan gum refers to a gellan gum consisting of polysaccharide molecules wherein the D-glucose residue in the repeating unit has about 50%±10% C6 substituted by acetyl group, preventing the molecules from forming network and therefore gelling. The gum is thus called highly acylated gellan gum due to its excellent viscoelasticity.

The lowly acetylated gellan gum according to the invention is a commercially available compound having an acetylation degree that is typical in the art. For example, the lowly acetylated gellan gum is a low acyl or deacylated gellan gum resulting from the removal of most acetyl groups by treating with a base at a high temperature. The gellan gum of this type exhibits superior gelling property.

The microorganisms present in the microbial alginates according to the invention are any microorganic species commonly used in the art with no specific limitation as long as the microbial alginate can be used as a hydrophilic gelling agent.

In the non-gelatin shell material of hard capsule or the non-gelatin shell of hard capsule obtained therefrom according to the invention, the typical content of the hydrophilic gelling agent is 0.01-18 parts by weight, preferably 0.1-18 parts by weight, more preferably 0.1-8.5 parts by weight, while the content of the adhesive is 62-96 parts by weight. Specifically, for example, the content of the hydrophilic gelling agent based on the total weight of the hard capsule shell material is 0.01 wt%-18 wt%, or 0.1 wt%-18 wt%, or 0.1 wt%-8.5 wt%.

### Adhesive

The adhesives according to the invention may be starch, dextrin, polyvinyl pyrrolidone (also known as povidone), carboxymethyl chitin, polyvinyl alcohol, sesbania gum, arabic gum, cassia gum, pullulan, pullulan acetate, elsinan, Indian gum, glucan, copolymer of vinyl pyrrolidone and vinyl acetate, hydroxyalkyl cellulose, carboxyalkyl cellulose or a combination thereof.

The adhesives are all commercially available compounds, the molecular weights of which are not limited as long as the object of the invention is not restricted thereby. Neither the polymerization mode nor the monomer ratio for the co-polymer is limited as long as the object of the invention is not restricted thereby.

As used herein, "cellulose" also encompasses metallic salts thereof, for example, alkali metallic salts, in particular sodium salts, such as sodium carboxymethyl cellulose.

Preferably, the adhesive is selected from high amylose, etherified starch, etherified high amylose, etherified high amylopectin, oxidized starch, oxidized high amylose, oxidized high amylopectin, esterified starch, esterified high amylose, esterified high amylopectin, hydroxyalkyl and/or carboxyalkyl cellulose or combinations thereof.

Preferably, the hydroxyalkyl cellulose is hydroxypropyl methyl cellulose, hydroxyethyl methyl cellulose, hydroxyethyl cellulose, hydroxypropyl cellulose or a combination thereof.

Examples of the carboxyalkyl cellulose include but are not limited to carboxymethyl cellulose, carboxyethyl cellulose or a combination thereof.

Preferably, the adhesive is hydroxyalkyl cellulose, carboxyalkyl cellulose, starch or a combination thereof. Specifically, for example, it is a composition of carboxyalkyl cellulose and starch. To the composition may also be added the following compounds: dextrin, polyvinyl pyrrolidone, carboxymethyl chitin, polyvinyl alcohol, sesbania gum, arabic gum, cassia gum, pullulan, pullulan acetate, elsinan, Indian gum, glucan, copolymer of vinyl pyrrolidone and vinyl acetate.

Preferably, the starch is selected from high amylose, etherified starch, etherified high amylose, etherified high amylopectin, oxidized starch, oxidized high amylose, oxidized high amylopectin, esterified starch, esterified high amylose, esterified high amylopectin.

A combination may be provided by combining hydroxyalkyl cellulose and starch at a weight ratio of, for example but not limited to, (12-5):1.

The typical content of the adhesive is 62-96 parts by weight, preferably 75-95 parts by weight, more preferably 75-95 parts by weight, while the typical content of the hydrophilic gelling agent is 0.01-18 parts by weight, preferably 0.1-18 parts by weight, more preferably 0.1-2 parts by weight.

### Water

The water used according to the invention is not specifically limited as long as the object of the invention is not restricted thereby. For example, it may be purified water, deionized water, distilled water, mineral water or a combination thereof.

The typical content of the water in the vegetal non-gelatin shell of hard capsule according to the invention is 3-10 parts by weight, while the typical content of the hydrophilic gelling agent is 0.01-18 parts by weight, preferably 0.1-18 parts by weight, more preferably 0.1-2 parts by weight.

### Plasticizer

A plasticizer may be introduced into the hard capsule shell material according to the invention as desired.

Representative plasticizers include but are not limited to glycerin, sorbitol, polyethylene glycol and mixtures thereof, ethylene glycol, ethyl acetate, 1,3-butylene glycol, 1,4-butylene glycol, xylitol, glyceryl diacetate, glyceryl triacetate, glyceryl monoacetate, mannitol, myoinositol, maltitol, glucose, polypropylene glycol or combinations thereof.

The typical content of the plasticizer is 0.01-5 parts by weight, preferably 0.1-4 parts by weight, while the typical content of the hydrophilic gelling agent is 0.01-18 parts by weight, preferably 0.1-18 parts by weight, more preferably 0.1-2 parts by weight.

Generally, the amount of the plasticizer that is used depends on the hardness of the product capsule, and is usually increased along with the increase of the amount of the hydrophilic gelling agent that is used.

### Co-gelling agent

A co-gelling agent may be introduced into the hard capsule shell according to the invention as desired.

Representative co-gelling agents include but are not limited to potassium chloride, calcium chloride, potassium phosphate, potassium citrate and mixtures thereof.

The typical content of the co-gelling agent is 0.01-5 parts by weight, preferably 0.1-4 parts by weight, while the typical content of the hydrophilic gelling agent is 0.01-18 parts by weight, preferably 0.1-18 parts by weight, more preferably 0.1-2 parts by weight.

Generally, the amount of the co-gelling agent is inversely proportional to that of the hydrophilic gelling agent.

### Non-gelatin shell material of hard capsule

The non-gelatin shell material of hard capsule according to the invention comprises the following components:
(A) 0.01-18 parts by weight of a hydrophilic non-gelatin gelling agent;
(B) 62-96 parts by weight of an adhesive;
(C) 3-10 parts by weight of water;
(D) 0-5 parts by weight of a co-gelling agent; and
(E) 0-5 parts by weight of a plasticizer,
wherein the sum of the weights of components (A) + (B) + (C) is 85wt%-100wt% of the total weight of the hard capsule shell material.

The main components of the non-gelatin shell material of hard capsule according to the invention include a hydrophilic gelling agent, an adhesive, a plasticizer and water. To the hard capsule shell material according to the invention may be added other additives as desired, with the content of the other additives being not more than 15 wt% of the total weight of the hard capsule shell material. The additives may be, for example, a preservative, an anti-oxidant, a pigment, a flavoring, a fragrance, etc. The components of the additives are not specifically limited as long as the object of the invention is not restricted thereby.

Representative preservatives include but are not limited to sodium benzoate, methyl p-hydroxy-benzoate, propyl p-hydroxy-benzoate and mixtures thereof. The typical content of the preservative based on the total weight of the hard capsule shell material is 0.001%-0.2%, preferably about 0.01%-0.19%. The amount of the preservative is generally proportional to that of the adhesive.

Representative anti-oxidants include but are not limited to propyl gallate, tea polyphenols, phytic acid, phosphatide, L-ascorbic acid and mixtures thereof. The typical content of the anti-oxidant based on the total weight of the hard capsule shell material is 0.001%-0.2%, preferably about 0.01%-0.19%.

Preferably, the hard capsule shell material according to the invention substantially consists of a hydrophilic gelling agent, an adhesive, an optional plasticizer, an optional co-gelling agent and water,
wherein the hydrophilic gelling agent is 0.01-18 parts by weight, the adhesive is 62-96 parts by weight, the water is 3-10 parts by weight, the plasticizer is 0-5 parts by weight, the co-gelling agent is 0-5 parts by weight, and wherein:
the gelling agent is selected from the group consisting of the following hydrophilic gelling agents: highly acetylated gellan gum, lowly acetylated gellan gum, κ-carrageenan, τ-carrageenan, β-carrageenan, agar, pectin, sodium alginate, xanthan gum, tragacanth gum, karaya gum, locust bean gum, furcellaran gum, tamarind gum, tara gum, sclerotium glucan, microbial alginate, carbomer or combinations thereof;
the adhesive is selected from the following adhesives: starch, dextrin, polyvinyl pyrrolidone, carboxymethyl chitin, polyvinyl alcohol, sesbania gum, arabic gum, cassia gum, pullulan, pullulan acetate, elsinan, Indian gum, glucan, copolymer of vinyl pyrrolidone and vinyl acetate, hydroxyalkyl cellulose, carboxyalkyl cellulose or combinations thereof;
the plasticizer is selected from the group consisting of the following plasticizers: the co-gelling agent is selected from the group consisting of the following co-gelling agent: potassium chloride, calcium chloride, potassium phosphate, potassium citrate or combinations thereof.

More preferably, the starch is selected from high amylose, etherified starch, etherified high amylose, etherified high amylopectin, oxidized starch, oxidized high amylose, oxidized high amylopectin, esterified starch, esterified high amylose, esterified high amylopectin or combinations thereof.

### Process of Preparation

The process for manufacturing the non-gelatin shell of hard capsule according to the invention comprises the following steps:
(a) providing 0.01-18 parts by weight of a hydrophilic non-gelatin gelling agent, 62-96 parts by weight of an adhesive, 0-5 parts by weight of an optional co-gelling agent and 0-5 parts by weight of an optional plasticizer, and mixing the foregoing components with water to give a gel solution of the dissolved components;
(b) using the gel solution obtained in step (a) to make a non-gelatin shell of hard capsule by gel-dipping or pressing; and
(c) drying the non-gelatin shell of hard capsule obtained in step (b) at 30-45°C to give a non-gelatin shell of hard capsule containing 3-10 parts by weight of water.

In one specific embodiment according to the invention, a non-gelatin shell material of hard capsule is prepared by mixing a hydrophilic gelling agent and an adhesive, after which water, a co-gelling agent and a plasticizer are added, and subsequently the resultant mixture is melted by heating to give the non-gelatin material. The corresponding steps are as follows: (a) mixing, while stirring, 0.01-5 parts by weight of a hydrophilic gelling agent, 10-40 parts by weight of an adhesive, 0.01-5 parts by weight of a plasticizer and 60-90 parts by weight of water to give a gel solution of the dissolved components, wherein 0.01 parts by weight of a defoamer such as glycerin polyoxypropylene ether, glycerin polyoxyethylene or a combination thereof, e.g. Paodi available from Northern Star Organic Chemical Engineering Co. located at Jiamusi, China, is preferably added during stirring;
(b) using the gel solution obtained in step (a) to make a non-gelatin shell of hard capsule by gel-dipping; and
(c) drying the non-gelatin shell of hard capsule obtained in step (b) at 30-45°C to give a non-gelatin shell of hard capsule containing 3%-10% water based on the total weight of the non-gelatin hard capsule.

In another preferred embodiment, it is possible to obtain a mixture first by mixing 1-6 parts by weight of a hydrophilic gelling agent, 5-40 parts by weight of an adhesive and 3-15 parts by weight of a plasticizer, after which the mixture is incorporated into 28.8-94 parts by weight of water, which has been heated to 75-95 °C, to give a liquid material by dissolving the gel evenly, and a hard capsule shell is obtained by gel-dipping from the liquid material which has been kept at certain temperature. After being dried, the capsule shell still contains some water which generally accounts for 3%-10% of a final product.

The process for manufacturing a non-gelatin shell of hard capsule according to the invention may be carried out using an existing equipment now used to manufacture gelatin shells of hard capsules. A desirable non-gelatin shell of hard capsule may be made by merely adjusting such process parameters as drying temperature, humidity, coating thickness, operation speed, mold size and the like according to the corresponding formulations, and no additional equipment is needed.

Compared with the prior art, the invention has the following principal advantages:
(1) Possible contamination caused by animal prion is avoided since the main gel material used to manufacture the capsule shell is a hydrophilic non-gelatin gel and any animal protein or fat is excluded.
(2) A plasticizer among those listed herein may be incorporated when necessary.
(3) Since a hydrophilic non-gelatin gelling agent is well soluble, the phase of water-absorbing and swelling as needed by the preparation of a gelatin capsule may be omitted, and thus the production time may be reduced.
(4) The tolerance of a non-gelatin hard capsule shell against a variety of humidities and temperatures makes it convenient to use, store and transport the shell. In contrast, a gelatin hard capsule shell has rather stringent requirement on humidity and temperature, so that its application is restricted.
(5) While sharing the advantages exhibited by a gelatin hard capsule shell, a non-gelatin hard capsule shell overcomes the disadvantages suffered by a gelatin hard capsule shell, such as a tendency to deform at a high temperature or when heated, a tendency to become brittle at a low temperature or at a low moisture content, no allowance to contain aldehyde-based medicaments, and the like. Furthermore, a hard capsule shell that is adapted to take effect instantly, be released slowly or controllably, or be gastric or intestinal soluble, may be made by tailoring the formulation thereof as desired.
(6) A non-gelatin hard capsule is soluble in cold water, while a gelatin hard capsule is swellable rather than soluble in cold water.
(7) In preferred embodiments according to the invention, a faster disintegrating rate and a better clarity may be achieved by using particular starch.

The compounds provided in the invention may be synthesized in conventional chemical conversion ways using commercially available starting materials.

Due to the disclosure of the invention, the other aspects of the invention will be obvious to those skilled in the art.

The invention will be explained in further detail with reference to the following specific examples. It is to be understood that these examples are provided only for the purpose of illustrating the invention without limiting the scope thereof. Generally, in the following examples, conventional conditions, such as those specified in "Beilstein Handbook of Organic Chemistry" (Chemical Industry Press, 1996) or suggested by manufacturers, will be used where no specific conditions are given for an experiment. Unless otherwise specified, all ratios and percentages are based on weight.

Unless otherwise defined or specified, all special and scientific terms used herein have the meanings known by those skilled in the art. In addition, any process or material similar or equivalent to those cited herein may be used in the invention.

### Example 1

| **Non-gelatin Shell Material 1 of Hard Capsule** | |
|---|---|
| Formula | Dosage (amount per 100 g of total weight) |
| τ-carrageenan | 2g |
| Hydroxypropyl methyl cellulose | 16 g |
| Hydroxypropyl starch | 3 g |
| Potassium chloride | 1g |
| Deionized water | 77 g |

Procedure: 2 g τ-carrageenan, 2 g hydroxypropyl starch and 1 g potassium chloride were added into 77 g deionized water, and the resultant mixture was heated to 100°C. After an even solution was obtained, 16 g hydroxypropyl methyl cellulose was added and stirred thoroughly. After defoamed while the temperature was kept constant, the solution was used to make a hard capsule shell by gel-dipping. After drying at 40°C, a clear, elastic and well-tasted non-gelatin shell of hard capsule was obtained with a moisture content of 2.4 g (10%). The starting materials listed above were all commercially available.

The break time of the capsule was 2 minutes as determined according to the standards on disintegrating time defined by "Pharmacopeia of the People's Republic of China"(2000).

### Example 2

| **Non-gelatin Shell Material 2 of Hard Capsule** | |
|---|---|
| Formula | Dosage (amount per 100 g of total weight) |
| Highly acetylated gellan gum (50% acetylated) | 0.5 g |
| Hydroxypropyl methyl cellulose | 22 g |
| Oxidized starch | 2g |
| Calcium chloride | 0.4 g |
| Deionized water | 75.1 g |

Procedure: 0.5 g highly acetylated gellan gum, 2 g oxidized starch and 0.4 g calcium chloride were added into 75.1 g deionized water, and the resultant mixture was heated to 100°C. After an even solution was obtained, 22 g hydroxypropyl methyl cellulose was added and stirred thoroughly. After defoamed while the temperature was kept constant, the solution was used to make a hard capsule shell by gel-dipping. After drying at 40°C, a clear, elastic and well-tasted non-gelatin shell of hard capsule was obtained with a moisture content of 0.8 g (3%). The starting materials listed above were all commercially available.

The break time of the capsule was 2 minutes as determined according to the standards on disintegrating time defined by "Pharmacopeia of the People's Republic of China"(2000).

### Example 3

| **Non-gelatin Shell Material 3 of Hard Capsule** | |
|---|---|
| Formula | Dosage (amount per 100 g of total weight) |
| Hydroxypropyl methyl cellulose | 25 g |
| κ-carrageenan | 1 g |
| Deionized water | 72 g |
| Glycerin | 2g |

Procedure: 1 g κ-carrageenan and 2 g glycerin were added into 72 g deionized water, and the resultant mixture was heated to 90°C. After an even solution was obtained, hydroxypropyl methyl cellulose was added and stirred thoroughly. After defoamed, the batch solution was cooled to 55°C, and then used to make a capsule shell by gel-dipping. The shell was dried at about 35°C to restrict the moisture content of the capsule membrane within 0.9 g (3%), and finally a clear and elastic non-gelatin shell of hard capsule was obtained. The starting materials listed above were all commercially available.

The break time of the capsule was 2.5 minutes as determined according to the standards on disintegrating time defined by "Pharmacopeia of the People's Republic of China"(2000).

### Example 4

| **Non-gelatin Shell Material 4 of Hard Capsule** | |
|---|---|
| Formula | Dosage (amount per 100 g of total weight) |
| κ-carrageenan | 0.5 g |
| Povidone | 9.5 g |
| Hydroxypropyl methyl cellulose | 18 g |
| Polyethylene glycol 4000 | 2g |
| Deionized water | 70 g |

Procedure: 0.5 g κ-carrageenan, 9.5 g povidone and 2 g polyethylene glycol (4000) were added into 70 g deionized water, and the resultant mixture was heated to 90°C. After an even solution was obtained, hydroxypropyl methyl cellulose was added and stirred thoroughly. After defoamed while the temperature was kept constant, the solution was used to make a capsule shell by gel-dipping. The shell was dried at about 30°C to restrict the moisture content of the capsule membrane within 3.3 g (10%), and finally a clear, elastic and well-tasted non-gelatin shell of hard capsule was obtained. The starting materials listed above were all commercially available.

The break time of the capsule was 2.5 minutes as determined according to the standards on disintegrating time defined by "Pharmacopeia of the People's Republic of China"(2000).

### Example 5

| **Non-gelatin Shell Material 5 of Hard Capsule** | |
|---|---|
| Formula | Dosage (amount per 100 g of total weight) |
| Gellan gum | 1 g |
| Hydroxypropyl methyl cellulose | 20 g |
| Polyethylene glycol 4000 | 0.8 g |
| Calcium chloride | 0.2 g |
| Deionized water | 78 g |

Procedure: 1 g gellan gum, 0.8 g polyethylene glycol and 0.2 g calcium chloride were added into 78 g deionized water, and the resultant mixture was heated to 100°C. After an even solution was obtained, 20 g hydroxypropyl methyl cellulose was added and stirred thoroughly. After defoamed while the temperature was kept constant, the solution was used to make a hard capsule shell by gel-dipping. After drying at 40°C, a clear, elastic and well-tasted non-gelatin shell of hard capsule was obtained with a moisture content of 0.7 g (3%). The starting materials listed above were all commercially available.

The break time of the capsule was 2.5 minutes as determined according to the standards on disintegrating time defined by "Pharmacopeia of the People's Republic of China"(2000).

### Example 6

| **Non-gelatin Shell Material 6 of Hard Capsule** | |
|---|---|
| Formula | Dosage (amount per 100 g of total weight) |
| κ-carrageenan | 1 g |
| Hydroxypropyl methyl cellulose | 20 g |
| Sodium carboxymethyl cellulose | 4g |
| Potassium citrate | 0.6 g |
| Deionized water | 75.5 g |

Procedure: 1 g κ-carrageenan, 4 g sodium carboxymethyl cellulose and 0.6 g potassium citrate were added into 75.5 g deionized water, and the resultant mixture was heated to 100°C. After an even solution was obtained, 20 g hydroxypropyl methyl cellulose was added and stirred thoroughly. After defoamed while the temperature was kept constant, the solution was used to make a hard capsule shell by gel-dipping. After drying at 40°C, a clear, elastic and well-tasted non-gelatin shell of hard capsule was obtained with a moisture content of 2.8 g (10%). The starting materials listed above were all commercially available.

The break time of the capsule was 3 minutes as determined according to the standards on disintegrating time defined by "Pharmacopeia of the People's Republic of China"(2000).

### Example 7

| **Non-gelatin Vegetal Shell Material 7 of Hard Capsule** | |
|---|---|
| Formula | Dosage (amount per 100 g of total weight) |
| Lowly acetylated gellan gum | 2g |
| Hydroxypropyl methyl cellulose | 18 g |
| Polyvinyl alcohol | 4g |
| Calcium chloride | 1 g |
| Deionized water | 75 g |

Procedure: 2 g lowly acetylated gellan gum, 4 g Polyvinyl alcohol and 1 g calcium chloride were added into 75 g deionized water, and the resultant mixture was heated to 100°C. After an even solution was obtained, 16 g hydroxypropyl methyl cellulose was added and stirred thoroughly. After defoamed while the temperature was kept constant, the solution was used to make a hard capsule shell by gel-dipping. After drying at 40°C, a clear, elastic and well-tasted non-gelatin shell of hard capsule was obtained with a moisture content of 2.8 g (10%). The starting materials listed above were all commercially available.

The break time of the capsule was 2.5 minutes as determined according to the standards on disintegrating time defined by "Pharmacopeia of the People's Republic of China"(2000).

### Example 8

### Non-gelatin Shell Material 8 of Hard Capsule

A non-gelatin shell material of hard capsule was obtained according to the process of Example 1, except that hydroxypropyl starch was substituted with the same amount of commercially available high amylopectin.

The break time of the capsule was 3 minutes as determined according to the standards on disintegrating time defined by "Pharmacopeia of the People's Republic of China"(2000).

### Comparative Example 1

A gelatin capsule was obtained according to the process of Example 1, except that the hydrophilic vegetal gelling agent and the adhesive were substituted with the same amount of gelatin. The product (a gelatin hard capsule) thus obtained was compared with the products obtained in Example 1 and 8, and the results were given as follows:
(1) The following Table 1 compared the disintegrating rates of the capsules:

**Table 1 Break Rates of the Capsules**

| Items | Non-gelatin Hard Capsule in Example 1 | Non-gelatin Hard Capsule in Example 8 | Gelatin Hard Capsule |
|---|---|---|---|
| Disintegrating Time of Capsule | 2 min | 3 min | 5 min |

The disintegrating time of the capsules was determined according to the standards on disintegrating time defined by "Pharmacopeia of the People's Republic of China"(2000).
(2) The following Table 1 compared the storing performance of the capsules:

**Table 2 Storing Performance of the Capsules**

| Items | Non-gelatin Hard Capsules in Example 1 and Example 8 | Gelatin Hard Capsule |
|---|---|---|
| Temperature: 0°C ; Humidity: 50% | Not getting brittle | Getting brittle |
| Temperature: 40°C ; Humidity: 50% | Not deformed | Deformed |

The storing time under the above storing conditions was one week.
(3) The following Table 1 compared the clarity of the capsules:

| Items | Non-gelatin Hard Capsule in Example 1 | Non-gelatin Hard Capsule in Example 8 | Gelatin Hard Capsule |
|---|---|---|---|
| Clarity | Relatively clear | Standard | Pale yellow |

The clarity was determined as follows:
90 ml water was added to a sample weighing 5.0g to make the sample swell, and then the sample was dissolved by heating in a water bath at 65-70°C. The solution was taken out, and additional water was added to make the volume of the solution to be 100 ml. An aliquot sample of 5 ml was taken from the solution and put into a 25ml Nessler tube. The turbidity of the aliquot sample was compared immediately with that of a control solution having the same volume (which was prepared as follows: 30 ml standard solution of sodium chloride was measured precisely and put into a 50 ml measuring flask to which 1 ml nitric acid and 1 ml silver nitrate were then added, the resultant solution was diluted by adding water to scale, the flask was placed in dark for 5 min, and the color of the solution might be adjusted using a caramel solution when necessary).

The control solution uses the non-gelatin hard capsule in Example 8. In order to improve the accuracy, each aliquot sample was measured three times. If the turbidity of an aliquot sample was lower than that of the control solution each time, the sample was considered "relatively clear".

All references mentioned in this disclosure are incorporated herein by reference, as if each of them would be incorporated herein by reference independently. In addition, it is to be appreciated that various changes or modifications can be made to the invention by those skilled in the art who have read the content taught above. These equivalents are intended to be included in the scope defined by the appended claims of the application.

## Claims

**1.** A non-gelatin shell material of hard capsule, comprising the following components:
(A) 0.01-18 parts by weight of a hydrophilic non-gelatin gelling agent;
(B) 62-96 parts by weight of an adhesive;
(C) 3-10 parts by weight of water;
(D) 0-5 parts by weight of a co-gelling agent; and
(E) 0-5 parts by weight of a plasticizer,
wherein the sum of the weights of components (A) + (B) + (C) is 85wt%-100wt% of the total weight of the hard capsule shell material.

**2.** A hard capsule shell material of Claim 1, wherein the adhesive of the component (B) is selected from the following adhesives: starch, dextrin, polyvinyl pyrrolidone, carboxymethyl chitin, polyvinyl alcohol, sesbania gum, arabic gum, cassia gum, pullulan, pullulan acetate, elsinan, Indian gum, glucan, copolymer of vinyl pyrrolidone and vinyl acetate, hydroxyalkyl cellulose, carboxyalkyl cellulose or combinations thereof.

**3.** A hard capsule shell material of Claim 1, wherein the adhesive of the component (B) is selected from starch, hydroxyalkyl cellulose or a combination thereof.

**4.** A hard capsule shell material of Claim 3, wherein the starch is selected from high amylose, etherified starch, etherified high amylose, etherified high amylopectin, oxidized starch, oxidized high amylose, oxidized high amylopectin, esterified starch, esterified high amylose, esterified high amylopectin or combinations thereof; and/or
the hydroxyalkyl cellulose is selected from hydroxypropyl methyl cellulose, hydroxyethyl methyl cellulose, hydroxypropyl cellulose or combinations thereof.

**5.** A hard capsule shell material of Claim 1, wherein the gelling agent of the component (A) is selected from the group consisting of the following hydrophilic gelling agents: highly acetylated gellan gum, lowly acetylated gellan gum, κ-carrageenan, τ-carrageenan, β-carrageenan, agar, pectin, sodium alginate, xanthan gum, tragacanth gum, karaya gum, locust bean gum, furcellaran gum, tamarind gum, tara gum, sclerotium glucan, microbial alginate, carbomer or combinations thereof;
the co-gelling agent of the component (D) is selected from the group consisting of the following co-gelling agents: potassium chloride, calcium chloride, potassium phosphate, potassium citrate or combinations thereof; and/or
the plasticizer of the component (E) is selected from the group consisting of the following plasticizers: glycerin, sorbitol, polyethylene glycol, ethylene glycol, ethyl acetate, 1,3-butylene glycol, 1,4-butylene glycol, xylitol, glyceryl diacetate, glyceryl triacetate, glyceryl monoacetate, mannitol, myoinositol, maltitol, glucose, polypropylene glycol or combinations thereof.

**7.** A hard capsule shell material of Claim 1, wherein the hard capsule shell material substantially consists of a hydrophilic gelling agent, an adhesive, an optional plasticizer, an optional co-gelling agent and water, wherein the hydrophilic gelling agent is 0.01-18 parts by weight, the adhesive is 62-96 parts by weight, the water is 3-10 parts by weight, the plasticizer is 0-5 parts by weight, the co-gelling agent is 0-5 parts by weight, and the total weight of the hydrophilic gelling agent, the adhesive and the water is 85wt%-100wt% of the total weight of the hard capsule shell material, and wherein:
the gelling agent is selected from the group consisting of the following hydrophilic gelling agents: highly acetylated gellan gum, lowly acetylated gellan gum, κ-carrageenan, τ-carrageenan, β-carrageenan, agar, pectin, sodium alginate, xanthan gum, tragacanth gum, karaya gum, locust bean gum, furcellaran gum, tamarind gum, tara gum, sclerotium glucan, microbial alginate, carbomer or combinations thereof;
the adhesive is selected from the following adhesives: starch, dextrin, polyvinyl pyrrolidone, carboxymethyl chitin, polyvinyl alcohol, sesbania gum, arabic gum, cassia gum, pullulan, pullulan acetate, elsinan, Indian gum, glucan, copolymer of vinyl pyrrolidone and vinyl acetate, hydroxyalkyl cellulose, carboxyalkyl cellulose or combinations thereof;
the plasticizer is selected from the group consisting of the following plasticizers: glycerin, sorbitol, polyethylene glycol and mixtures thereof, ethylene glycol, ethyl acetate, 1,3-butylene glycol, 1,4-butylene glycol, xylitol, glyceryl diacetate, glyceryl triacetate, glyceryl monoacetate, mannitol, myoinositol, maltitol, glucose, polypropylene glycol or combinations thereof; and/or
the co-gelling agent is selected from the group consisting of the following co-gelling agents: potassium chloride, calcium chloride, potassium phosphate, potassium citrate or combinations thereof.

**8.** A hard capsule shell material of Claim 7, wherein the starch is selected from high amylose, etherified starch, etherified high amylose, etherified high amylopectin, oxidized starch, oxidized high amylose, oxidized high amylopectin, esterified starch, esterified high amylose, esterified high amylopectin or combinations thereof.

**9.** A hard capsule made from the non-gelatin shell material of hard capsule of Claim 1.

**10.** A process for manufacturing a non-gelatin shell of hard capsule, comprising the following steps:
(a) providing 0.01-18 parts by weight of a hydrophilic non-gelatin gelling agent, 62-96 parts by weight of an adhesive, 0-5 parts by weight of an optional co-gelling agent and 0-5 parts by weight of an optional plasticizer, and mixing the foregoing components with water to give a gel solution of the dissolved components;
(b) using the gel solution obtained in step (a) to make a non-gelatin shell of hard capsule by gel-dipping or pressing; and
(c) drying the non-gelatin shell of hard capsule obtained in step (b) at 30-45°C to give a non-gelatin shell of hard capsule containing 3-10 parts by weight of water.
